(19) **Europäisches Patentamt European Patent Office Office européen des brevets**

(11) **EP 2 191 843 A1**

(12) # EUROPEAN PATENT APPLICATION

(43) Date of publication:
**02.06.2010 Bulletin 2010/22**

(51) Int Cl.:
*A61K 39/00* *(2006.01)*      *A61K 31/00* *(2006.01)*
*A61P 35/00* *(2006.01)*

(21) Application number: **08291119.9**

(22) Date of filing: **28.11.2008**

(84) Designated Contracting States:
**AT BE BG CH CY CZ DE DK EE ES FI FR GB GR HR HU IE IS IT LI LT LU LV MC MT NL NO PL PT RO SE SI SK TR**
Designated Extension States:
**AL BA MK RS**

(71) Applicant: **Sanofi-Aventis**
**75013 Paris (FR)**

(72) Inventor: **The designation of the inventor has not yet been filed**

(74) Representative: **Bouvet, Philippe et al**
**Patent Department ADF2**
**174 Avenue de France**
**75635 Paris Cedex 13 (FR)**

(54) **Antitumor combinations containing antibodies recognizing specifically CD38 and cyclophosphamide**

(57) Pharmaceutical composition comprising an antibody specifically recognizing CD38 and cyclophosphamide.

EP 2 191 843 A1

**Description**

The present invention relates to combinations of monoclonal antibodies directed against CD38 and cyclophosphamide known under the trade-name cytoxan (or neosar) which are therapeutically useful in the treatment of neoplastic diseases.

CD38 is a 45 kD type II transmembrane glycoprotein with a long C-terminal extracellular domain and a short N-terminal cytoplasmic domain. The CD38 protein is a bifunctional ectoenzyme that can catalyze the conversion of $NAD^+$ into cyclic ADP-ribose (cADPR) and also hydrolyze cADPR into ADP-ribose. CD38 is upregulated and has been implicated in many hematopoietic malignancies.

Monoclonal antibodies 38SB13, 38SB18, 38SB19, 38SB30, 38SB31, and 38SB39, which specifically recognize CD38, are described in PCT application WO2008/047242. Said anti-CD38 antibodies are capable of killing $CD38^+$ cells by three different cytotoxic mechanisms, induction of apoptosis, antibody-dependent cell-mediated cytotoxicity (ADCC), and complement-dependent cytotoxicity (CDC). In addition, these antibodies are able to directly induce apoptosis of $CD38^+$ cells, even without the presence of stroma cells or stroma-derived cytokines. Cyclophosphamide is an alkylating agent used in chemotherapy. There is still a need for novel and efficacious medicaments which can be used in cancer therapy.

It has now been found, and for this invention, that the efficacy of the humanized anti-CD38 antibodies may be considerably improved when it is administered in combination with at least one substance which is therapeutically useful in anticancer treatments and has a mechanism identical to or different from the one of the humanized anti-CD38 antibodies and which is limited in the present invention to cyclophosphamide.

The term "antibody" is used herein in the broadest sense and specifically covers monoclonal antibodies (including full length monoclonal antibodies) of any isotype such as IgG, IgM, IgA, IgD and IgE, polyclonal antibodies, multispecific antibodies, chimeric antibodies, and antibody fragments. A typical IgG antibody is comprised of two identical heavy chains and two identical light chains that are joined by disulfide bonds. Each heavy and light chain contains a constant region and a variable region. Each variable region contains three segments called "complementarity-determining regions" ("CDRs") or "hypervariable regions", which are primarily responsible for binding an epitope of an antigen. They are usually referred to as CDR1, CDR2, and CDR3, numbered sequentially from the N-terminus. The more highly conserved portions of the variable regions outside of the CDRs are called the "framework regions".

As used herein, "$V_H$" or "VH" refers to the variable region of an immunoglobulin heavy chain of an antibody, including the heavy chain of an Fv, scFv, dsFv, Fab, Fab' or F(ab')2 fragment. Reference to "$V_L$" or "VL" refers to the variable region of the immunoglobulin light chain of an antibody, including the light chain of an Fv, scFv, dsFv, Fab, Fab' or F(ab')2 fragment.

The 38SB13 antibody comprises at least one heavy chain having an amino acid sequence consisting of SEQ ID NO: 50 and at least one light chain having an amino acid sequence consisting of SEQ ID NO: 38, said heavy chain comprising three sequential CDRs having amino acid sequences consisting of SEQ ID NOS: 1, 2, and 3, and said light chain comprising three sequential CDRs having amino acid sequences consisting of SEQ ID NOS: 4, 5, and 6.

The 38SB18 antibody comprises at least one heavy chain having an amino acid sequence consisting of SEQ ID NO: 52 and at least one light chain having an amino acid sequence consisting of SEQ ID NO: 40, said heavy chain comprising three sequential CDRs having amino acid sequences consisting of SEQ ID NOS: 7, 8, and 9, and said light chain comprising three sequential CDRs having amino acid sequences consisting of SEQ ID NOS: 10, 11, and 12.

The 38SB19 antibody comprises at least one heavy chain having an amino acid sequence consisting of SEQ ID NO: 54 and at least one light chain having an amino acid sequence consisting of SEQ ID NO: 42, said heavy chain comprising three sequential CDRs having amino acid sequences consisting of SEQ ID NOS: 13, 14, and 15, and said light chain comprising three sequential CDRs having amino acid sequences consisting of SEQ ID NOS: 16, 17, and 18.

The 38SB30 antibody comprises at least one heavy chain having an amino acid sequence consisting of SEQ ID NO: 56 and at least one light chain having an amino acid sequence consisting of SEQ ID NO: 44, said heavy chain comprising three sequential CDRs having amino acid sequences consisting of SEQ ID NOS: 19, 20, and 21, and said light chain comprising three sequential CDRs having amino acid sequences consisting of SEQ ID NOS: 22, 23, and 24.

The 38SB31 antibody comprises at least one heavy chain having an amino acid sequence consisting of SEQ ID NO: 58 and at least one light chain having an amino acid sequence consisting of SEQ ID NO: 46, said heavy chain comprising three sequential CDRs having amino acid sequences consisting of SEQ ID NOS: 25, 26, and 27, and said light chain comprising three sequential CDRs having amino acid sequences consisting of SEQ ID NOS: 28, 29, and 30.

The 38SB39 antibody comprises at least one heavy chain having an amino acid sequence consisting of SEQ ID NO: 60 and at least one light chain having an amino acid sequence consisting of SEQ ID NO: 48, said heavy chain comprising three sequential CDRs having amino acid sequences consisting of SEQ ID NOS: 31, 32, and 33, and said light chain comprising three sequential CDRs having amino acid sequences consisting of SEQ ID NOS: 34, 35, and 36.

The hybridoma cell lines producing the 38SB13, 38SB18, 38SB19, 38SB30, 38SB31, and 38SB39 murine anti-CD38 antibodies have been deposited at the American Type Culture Collection (10801 University Bld, Manassas, VA, 20110-2209, USA), on June 21, 2006, under the deposit numbers PTA-7667, PTA-7669, PTA-7670, PTA-7666, PTA-7668, and PTA-7671, respectively (as described in WO2008/047242).

The term "humanized antibody", as used herein, refers to a chimeric antibody which contain minimal sequence derived from non-human immunoglobulin. The goal of humanization is a reduction in the immunogenicity of a xenogenic antibody, such as a murine antibody, for introduction into a human, while maintaining the full antigen binding affinity and specificity of the antibody. Humanized antibodies, or antibodies adapted for non-rejection by other mammals, may be produced using several technologies such as resurfacing and CDR grafting. As used herein, the resurfacing technology uses a combination of molecular modelling, statistical analysis and mutagenesis to alter the non-CDR surfaces of antibody variable regions to resemble the surfaces of known antibodies of the target host. The CDR grafting technology involves substituting the complementarity determining regions of, for example, a mouse antibody, into a human framework domain, e.g., see W0 92/22653. Humanized chimeric antibodies preferably have constant regions and variable regions other than the complementarity determining regions (CDRs) derived substantially or exclusively from the corresponding human antibody regions and CDRs derived substantially or exclusively from a mammal other than a human.

Strategies and methods for the resurfacing of antibodies, and other methods for reducing immunogenicity of antibodies within a different host, are disclosed in US Patent 5,639,641, which is hereby incorporated in its entirety by reference. Antibodies can be humanized using a variety of other techniques including CDR-grafting (EP 0 239 400; WO 91/09967; U.S. Pat. Nos. 5,530,101; and 5,585,089), veneering or resurfacing (EP 0 592 106; EP 0 519 596; Padlan E. A., 1991, Molecular Immunology 28(4/5): 489-498; Studnicka G. M. et al., 1994, Protein Engineering, 7(6): 805-814; Roguska M.A. et al., 1994, PNAS, 91: 969-973), chain shuffling (U.S. Pat. No. 5,565,332), and identification of flexible residues (PCT/US2008/074381). Human antibodies can be made by a variety of methods known in the art including phage display methods. See also U.S. Pat. Nos. 4,444,887, 4,716,111, 5,545,806, and 5,814,318; and international patent application publication numbers WO 98/46645, WO 98/50433, WO 98/24893, WO 98/16654, WO 96/34096, WO 96/33735, and WO 91/10741 (said references incorporated by reference in their entireties).

The anti-CD38 antibodies of the pharmaceutical combination of the present invention are humanized antibodies which recognize CD38 and kill CD38$^+$ cells by apoptosis, ADCC, and CDC. In a further embodiment, the humanized antibodies of the invention are capable of killing said CD38$^+$ cells by apoptosis even in the absence of stroma cells or stroma-derived cytokines.

A preferred embodiment of such a humanized antibody is a humanized 38SB13, 38SB18, 38SB19, 38SB30, 38SB31, or 38SB39 antibody, or an epitope-binding fragment thereof.

The CDRs of the 38SB13, 38SB18, 38SB19, 38SB30, 38SB31, and 38SB39 antibodies are identified by modelling and their molecular structures have been predicted. Thus, in one embodiment, this invention provides humanized antibodies or epitope-binding fragment thereof comprising one or more CDRs having an amino acid sequence selected from the group consisting of SEQ ID NOS: 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, and 36. In a preferred embodiment, a humanized version of 38SB13 is provided, which comprises at least one heavy chain and at least one light chain, wherein said heavy chain comprises three sequential complementarity-determining regions having amino acid sequences represented by SEQ ID NOS: 1, 2, and 3, and wherein said light chain comprises three sequential complementarity-determining regions having amino acid sequences represented by SEQ ID NOS: 4, 5, and 6. In another preferred embodiment, a humanized version of 38SB18 is provided, which comprises at least one heavy chain and at least one light chain, wherein said heavy chain comprises three sequential complementarity-determining regions having amino acid sequences represented by SEQ ID NOS: 7, 8, and 9, and wherein said light chain comprises three sequential complementarity-determining regions having amino acid sequences represented by SEQ ID NOS: 10, 11, and 12. In another preferred embodiment, a humanized version of 38SB19 is provided, which comprises at least one heavy chain and at least one light chain, wherein said heavy chain comprises three sequential complementarity-determining regions having amino acid sequences represented by SEQ ID NOS: 13, 14, and 15, and wherein said light chain comprises three sequential complementarity-determining regions having amino acid sequences represented by SEQ ID NOS: 16, 17, and 18. In another preferred embodiment, a humanized version of 38SB30 is provided, which comprises at least one heavy chain and at least one light chain, wherein said heavy chain comprises three sequential complementarity-determining regions having amino acid sequences represented by SEQ ID NOS: 19, 20, and 21, and wherein said light chain comprises three sequential complementarity-determining regions having amino acid sequences represented by SEQ ID NOS: 22, 23, and 24. In another preferred embodiment, a humanized version of 38SB31 is provided, which comprises at least one heavy chain and at least one light chain, wherein said heavy chain comprises three sequential complementarity-determining regions having amino acid sequences represented by SEQ ID NOS: 25, 26, and 27, and wherein said light chain comprises three sequential complementarity-determining regions having amino acid sequences represented by SEQ ID NOS: 28, 29, and 30. In another preferred embodiment, a humanized version of 38SB39 is provided, which comprises at least one heavy chain and at least one light chain, wherein said heavy chain comprises three sequential complementarity-determining regions having amino acid sequences represented by SEQ ID NOS: 31, 32, and 33, and wherein said light chain comprises three sequential complementarity-determining regions having amino acid sequences represented by SEQ ID NOS: 34, 35, and 36.

In one embodiment, this invention provides humanized antibodies or fragments thereof which comprise a $V_H$ having an amino acid sequence selected from the group of SEQ ID NOS: 66 and 72. In a preferred embodiment, a humanized 38SB19 antibody is provided which comprises a $V_H$ having an amino acid sequence represented by SEQ ID NO: 66. In another preferred embodiment, a humanized 38SB31 antibody is provided which comprises a $V_H$ having an amino acid sequence represented by SEQ ID NO: 72.

In another embodiment, this invention provides humanized antibodies or fragments thereof which comprise a $V_L$ having an amino acid sequence selected from the group of SEQ ID NOS: 62, 64, 68, and 70. In a preferred embodiment, a humanized 38SB19 antibody is provided which comprises a $V_L$ having an amino acid sequence chosen from the group of SEQ ID NOS: 62 and 64. In another preferred embodiment, a humanized 38SB31 antibody is provided which comprises a $V_L$ having an amino acid sequence chosen from the group of SEQ ID NOS: 68 and 70.

Each of the humanized versions of the 38SB13, 38SB18, 38SB19, 38SB30, 38SB31, and 38SB39 antibodies has been shown to be particularly advantageous as an anticancer agent. The preparation, physical properties and beneficial pharmacological properties thereof are described in WO 2008/047242, which is incorporated by reference herein in its entirety. Generally, the doses used for treating human beings, which depend on factors distinctive to the subject to be treated, are between 1 and 150 mg/kg administered orally or between 1 and 150 mg/kg administered intravenously.

Cyclophosphamide (cytoxan®) is a nitrogen mustard alkylating agent used to treat various cancers. It is a pro-drug that is converted in the liver to active forms that have chemotherapeutic activity. As an alkylating agent, cyclophosphamide prevents cell division primarily by crosslinking DNA strands, ultimately resulting in cell death. Cyclophosphamide is available in both oral and parental formulations.

One aspect of the invention is a pharmaceutical composition comprising an anti-CD38 antibody in combination with at least cyclophosphamide. Since the activity of the products depends on the doses used, it is thus possible to use lower doses and to increase the activity while decreasing the toxicity phenomena. The improved efficacy of a combination according to the invention may be demonstrated by determination of the therapeutic synergy. A combination manifests therapeutic synergy if it is therapeutically superior to the best agent of the study used alone at its maximum tolerated dose or at its highest dose tested when toxicity cannot be reached in the animal species.

This efficacy may be quantified, for example, by the $\log_{10}$ cell kill, which is determined according to the following formula:

$$\log_{10} \text{ cell kill} = \text{T-C (days)}/3.32 \times T_d$$

in which T - C represents the tumor growth delay, which is the median time in days for the tumors of the treated group (T) and the tumors of the control group (C) to have reached a predetermined value (1 g for example), and $T_d$ represents the time in days needed for the volume of the tumor to double in the control animals [T.H. Corbett et al., Cancer, 40: 2660-2680 (1977); F.M. Schabel et al., Cancer Drug Development, Part B, Methods in Cancer Research, 17: 3-51, New York, Academic Press Inc. (1979)]. A product is considered to be active if $\log_{10}$ cell kill is greater than or equal to 0.7. A product is considered to be very active if $\log_{10}$ cell kill is greater than or equal to 2.8.

In some cases, when treatment duration is over 10 days and differs between the 2 compounds evaluated in the combination, a log cell kill net is calculated:

$$\log_{10} \text{ cell kill net} = \text{(T-C)} - \text{treatment duration}/3.32 \times T_d.$$

Activity is declared when log cell kill net is greater than or equal to 0.

The combination will manifest therapeutic synergy when the $\log_{10}$ cell kill is greater than the value of the $\log_{10}$ cell kill of the best constituent administered alone at its maximum tolerated dose or at its highest dose tested.

The efficacy of the combinations on solid tumors may be determined experimentally in the following manner:

The animals subjected to the experiment, generally mice, are subcutaneously grafted bilaterally with 30 to 60 mg of a tumor fragment on day 0. The animals bearing tumors are randomized based on their tumor size before being subjected to the various treatments and controls. Chemotherapy begins when tumors have reached a predetermined size after grafting, depending on the type of tumor, and the animals are observed every day. The different animal groups are weighed daily during treatment until the maximum weight loss is reached and subsequent full weight recovery has occurred. The groups are then weighed once or twice a week until the end of the trial.

The tumors are measured 1 to 5 times a week, depending on the tumor doubling time, until the tumor reaches approximately 2 g, or until the animal dies (if this occurs before the tumor reaches 2 g). The animals are necropsied immediately after euthanasia or death.

The antitumor activity is determined in accordance with the different parameters recorded.

Results obtained with combinations of hu38SB19 and cyclophosphamide used at their optimal doses are indicated hereunder as examples.

The present invention also relates, therefore, to pharmaceutical compositions containing the combinations according to the invention.

The constituents of which the combination are composed may be administered simultaneously, semi-simultaneously, separately, or spaced out over a period of time so as to obtain the maximum efficacy of the combination; it being possible for each administration to vary in its duration from a rapid administration to a continuous perfusion.

As a result, for the purposes of the present invention, the combinations are not exclusively limited to those which are obtained by physical association of the constituents, but also to those which permit a separate administration, which can be simultaneous or spaced out over a period of time.

The compositions according to the invention are preferably compositions which can be administered parentally. However, these compositions may be administered orally, subcutaneously or intraperitoneally in the case of localized regional therapies.

The compositions for parental administration are generally pharmaceutically acceptable, sterile solutions or suspensions which may optionally be prepared as required at the time of use. For the preparation of non-aqueous solutions or suspensions, natural vegetable oils such as olive oil, sesame oil or liquid petroleum or injectable organic esters such as ethyl oleate may be used. The sterile aqueous solutions can consist of a solution of the product in water. The aqueous solutions are suitable for intravenous administration provided the pH is appropriately adjusted and the solution is made isotonic, for example with a sufficient amount of sodium chloride or glucose. The sterilization may be carried out by heating or by any other means which does not adversely affect the composition. The combinations may also take the form of liposomes or the form of an association with carriers as cyclodextrins or polyethylene glycols.

The compositions for oral, subcutaneous or intraperitoneal administration are preferably aqueous suspensions or solutions.

In the combinations according to the invention, the application of the constituents of which may be simultaneous, separate or spaced out over a period of time, it is especially advantageous for the amount of humanized anti-CD38 antibody to represent from 10 to 90 % by weight of the combination, it being possible for this content to vary in accordance with the nature of the associated substance, the efficacy sought and the nature of the cancer to be treated.

The combinations according to the invention are especially useful in the treatment of several types of cancers including (but not limited to) the following: carcinomas and adenocarcinomas, including that of the bladder, breast, colon, head-and-neck, prostate, kidney, liver, lung, ovary, pancreas, stomach, cervix, thyroid and skin, and including squamous cell carcinoma ; hematopoietic tumors of lymphoid lineage, including multiple myeloma, leukemia, acute and chronic lymphocytic (or lymphoid) leukemia, acute and chronic lymphoblastic leukemia, B-cell lymphoma, T-cell lymphoma, non-Hodgkin lymphoma (e.g. Burkitt's lymphoma) ; hematopoietic tumors of myeloid lineage, including acute and chronic myelogenous (myeloid or myelocytic) leukemias, and promyelocytic leukemia; tumors of mesenchymal origin, including fibrosarcoma, osteosarcoma and rhabdomyosarcoma; tumors of the central and peripheral nervous system, including astrocytoma, neuroblastoma, glioma, and schwannomas; and other tumors, including melanoma, teratocarcinoma, xeroderma pigmentosum, keratoacanthoma, and seminoma, and other cancers yet to be determined in which CD38 is expressed. They are mainly useful for treating leukemia, lymphoma and cancers resistant to the commonly used anticancer agents as the anti-CD38 antibodies of the invention have a unique mechanism of action.

Thus, the invention also encompasses the use of the above combinations for the manufacture of a medicament for the treatment of cancer.

## Example:

In this example, the effectiveness of an anti-CD38 antibody/cyclophosphamide combination of the invention for tumor growth inhibition was demonstrated in vivo.

The selected tumor model was a transplantable human Burkitt's lymphoma cell line, the Namalwa model, implanted in SCID mice.

Hu38SB19 was formulated in glucose 5 % in water. Hu38SB19, as single agent, was administered intravenously, twice a week, between days 11 and 18 post-tumor implantation. In the combination arm, hu38SB19 treatment was pursued until day 35 (except for the highest dose of the combination for which treatment was stopped when toxicity was reached on day 18).

Cyclophosphamide was formulated in glucose 5 % in water. Cyclophosphamide was administered intravenously, once per day, on day 11 and 15 after tumor implantation.

The results of the experiment are reported in Table 1.

Tumor doubling time = 1.9 days.

The following end points have been used:

- Toxicity was declared at dosages inducing $\geq$ 20 % body weight loss or $\geq$ 10 % drug death,
- Antitumor efficacy was determinated by calculating $\log_{10}$ cell kill gross = (T-C) / [3.32 x (tumor doubling time in days)] (T meaning the median time of the treated mice to reach 1000 mg and C the median time (15.7 days) of the control mice to reach the same size; tumor-free survivors are excluded from these calculations and are tabulated separately). No antitumor activity was declared for log cell kill gross < 0.7, and the treatment was declared highly active for log cell kill $\geq$ 2.8
- Tumor Free Survivors (TFS): correspond to complete regression below the limit of palpation (63 mg) for the entire duration of the study (>100 days post last treatment).
- Therapeutic Synergism: a combination has therapeutic synergism if it is more active than the best single agent of the study (by at least 1 log cell kill).

Toxicity for cyclophosphamide alone was observed at a dose of 286.1 mg/kg/injection, with 3 deaths out of 5 mice (60 %). Thus the highest nontoxic dose (HNTD) for cyclophosphamide was 177.4 mg/kg/inj (total injected dose = 354.8 mg/kg). The 177.4 mg/kg/inj dose was found to be highly active with a log cell kill gross of 4.8.

Regarding hu38SB19, the product was well tolerated at a dose of 40 mg/kg/inj (total dose of 120.0 mg/kg). No toxicity was observed, which can be explained by the lack of cross-reactivity of the antibody with murine CD38. The log cell kill was 0.4, indicating that hu38DB19 was not active under these conditions.

The combination of cyclophosphamide at 286.1 mg/kg/inj and hu38SB19 at 40 mg/kg/inj was toxic, with 3 drug-related deaths out of 6 animals (50 %), i.e. very similar to what was observed with cyclophosphamide alone at the same dose. The dose of 177.4 mg/kg/inj of cyclophosphamide with 40 mg/kg/inj of hu38SB19 was considered to be the HNTD. This dose displayed a log cell kill of 20.0 and 1/6 TFS (day 196) and was thus considered highly active. Even the lower doses of 110.0 mg/kg/inj and 68.2 mg/kg/inj of cyclophosphamide with 40 mg/kg/inj of hu38SB19 were highly active, with log cell kill gross of 15.7 and 6.9, respectively. Remarkably, the antitumor activity of the combination, at all three dose levels evaluated, was greater by more than one log cell kill than the one observed for the best agent, cyclophosphamide (4.8 log cell kill at the HNTD). We conclude that this combination shows a therapeutic synergism. This therapeutic synergism is also clearly established using the log cell kill net value taking into account the treatment duration (16 log cell kill net for the combination versus 4 log cell kill net for cyclophosphamide alone).

Table I: Combination of hu38SB19 and cyclophosphamide (CPA) against advanced human Burkitt's lymphoma Namalwa implanted in SCID female mice.

| agent, schedule and dose in mg/kg/inj (total dose) | | % BWC at nadir (day) | T-C in days (1000 mg) | $\log_{10}$ cell kill gross /net | Comments |
|---|---|---|---|---|---|
| hu38SB19, IV Day 11-35, twice a week 40.0 (120.0)* | CPA, IV Day 11, 15 - | +5.4 (19) | 2.5 | 0.4/-0.9 | HDT - inactive |
| - | 286.1 (572.2) | -21.2 (20) | - | - | Toxic 3/5 deaths |
| - | 177.4 (354.8) | -11.1 (19) | 30.1 | 4.8/4.0 | HNTD, highly active |
| - | 110.0 (220.0) | -5.2 (17) | 19.8 | 3.1/2.3 | Highly active |
| - | 68.2 (136.4) | -3.8(18) | 9.8 | 1.6/0.8 | Active |
| 40.0 (120.0)* | 286.1 (572.2) | -21.7 (21) | - | - | Toxic 3/6 deaths |
| 40.0 (320.0) | 177.4 (354.8) | -8.0 (16) | 126.1 | 20.0/16.0 | HNTD, highly active 1/6 TFS |
| 40.0 (320.0) | 110.0 (220.0) | -4.2 (17) | 99.3 | 15.7/11.8 | Highly active |

(continued)

| agent, schedule and dose in mg/kg/inj (total dose) | | % BWC at nadir (day) | T-C in days (1000 mg) | log$_{10}$ cell kill gross /net | Comments |
|---|---|---|---|---|---|
| 40.0 (320.0) | 68.2 (136.4) | -4.6 (29) | 43.7 | 6.9/3.0 | Highly active |
| Tumor doubling time = 1.9 days. Median tumor size at start of therapy = 142-149 mg. Time for median tumor to reach 1000 mg = 15.7 days. Formulations: hu38SB19 and CPA = glucose 5 % in water. BWC = body weight change, T-C = tumor growth delay, HNTD = highest nontoxic dose, HDT = highest dose tested, CPA = cyclophosphamide, TFS = tumor free survivors, IV = intravenous. * Treament was stopped on day 18. | | | | | |

SEQUENCE LISTING

<110> SANOFI-AVENTIS

<120> ANTITUMOR COMBINATIONS CONTAINING ANTIBODIES RECOGNIZING
SPECIFICALLY CD38 AND CYCLOPHOSPHAMIDE

<130> FR2008-114

<160> 80

<170> PatentIn version 3.3

<210> 1
<211> 5
<212> PRT
<213> Mus sp.

<400> 1

Ser Tyr Gly Met Asn
1               5

<210> 2
<211> 17
<212> PRT
<213> Mus sp.

<400> 2

Trp Ile Asn Thr Tyr Thr Gly Glu Pro Thr Tyr Ala Asp Asp Phe Lys
1               5                   10                  15

Gly

<210> 3
<211> 5
<212> PRT
<213> Mus sp.

<400> 3

Arg Gly Phe Ala Tyr
1               5

<210> 4
<211> 15
<212> PRT
<213> Mus sp.

<400> 4

Arg Ala Ser Glu Ser Val Glu Ile Tyr Gly Asn Gly Phe Met Asn
1               5                   10                  15

<210> 5
<211> 7
<212> PRT
<213> Mus sp.

<400> 5

8

```
Arg Ala Ser Asn Leu Glu Ser
1               5

<210>   6
<211>   9
<212>   PRT
<213>   Mus sp.

<400>   6

Gln Gln Ile Asn Glu Asp Pro Phe Thr
1               5

<210>   7
<211>   5
<212>   PRT
<213>   Mus sp.

<400>   7

Asn Ser Gly Met Asn
1               5

<210>   8
<211>   17
<212>   PRT
<213>   Mus sp.

<400>   8

Trp Ile Asn Thr Tyr Thr Gly Glu Pro Thr Tyr Ala Asp Asp Phe Lys
1               5                   10                  15

Gly

<210>   9
<211>   5
<212>   PRT
<213>   Mus sp.

<400>   9

Arg Gly Phe Val Tyr
1               5

<210>   10
<211>   15
<212>   PRT
<213>   Mus sp.

<400>   10

Arg Ala Ser Glu Ser Val Ala Ile Tyr Gly Asn Ser Phe Leu Lys
1               5                   10                  15

<210>   11
<211>   7
<212>   PRT
<213>   Mus sp.
```

<400> 11

Arg Ala Ser Asn Leu Glu Ser
1               5


<210> 12
<211> 9
<212> PRT
<213> Mus sp.

<400> 12

Gln Gln Ile Asn Glu Asp Pro Tyr Thr
1               5


<210> 13
<211> 5
<212> PRT
<213> Mus sp.

<400> 13

Asp Tyr Trp Met Gln
1               5


<210> 14
<211> 17
<212> PRT
<213> Mus sp.

<400> 14

Thr Ile Tyr Pro Gly Asp Gly Asp Thr Gly Tyr Ala Gln Lys Phe Lys
1               5                   10                  15

Gly


<210> 15
<211> 11
<212> PRT
<213> Mus sp.

<400> 15

Gly Asp Tyr Tyr Gly Ser Asn Ser Leu Asp Tyr
1               5                   10


<210> 16
<211> 11
<212> PRT
<213> Mus sp.

<400> 16

Lys Ala Ser Gln Asp Val Ser Thr Val Val Ala
1               5                   10


<210> 17
<211> 7
<212> PRT

```
<213>  Mus sp.

<400>  17

Ser Ala Ser Tyr Arg Tyr Ile
1               5


<210>  18
<211>  9
<212>  PRT
<213>  Mus sp.

<400>  18

Gln Gln His Tyr Ser Pro Pro Tyr Thr
1               5


<210>  19
<211>  5
<212>  PRT
<213>  Mus sp.

<400>  19

Gly Ser Trp Met Asn
1               5


<210>  20
<211>  17
<212>  PRT
<213>  Mus sp.

<400>  20

Arg Ile Tyr Pro Gly Asp Gly Asp Ile Ile Tyr Asn Gly Asn Phe Arg
1               5                   10                  15

Asp


<210>  21
<211>  10
<212>  PRT
<213>  Mus sp.

<400>  21

Trp Gly Thr Phe Thr Pro Ser Phe Asp Tyr
1               5                   10


<210>  22
<211>  11
<212>  PRT
<213>  Mus sp.

<400>  22

Lys Ala Ser Gln Asp Val Val Thr Ala Val Ala
1               5                   10


<210>  23
```

<211> 7
<212> PRT
<213> Mus sp.

<400> 23

Ser Ala Ser His Arg Tyr Thr
1               5


<210> 24
<211> 9
<212> PRT
<213> Mus sp.

<400> 24

Gln Gln His Tyr Thr Thr Pro Thr Thr
1               5


<210> 25
<211> 5
<212> PRT
<213> Mus sp.

<400> 25

Ser Tyr Thr Leu Ser
1               5


<210> 26
<211> 17
<212> PRT
<213> Mus sp.

<400> 26

Thr Ile Ser Ile Gly Gly Arg Tyr Thr Tyr Tyr Pro Asp Ser Val Glu
1               5                   10                  15

Gly


<210> 27
<211> 8
<212> PRT
<213> Mus sp.

<400> 27

Asp Phe Asn Gly Tyr Ser Asp Phe
1               5


<210> 28
<211> 11
<212> PRT
<213> Mus sp.

<400> 28

Lys Ala Ser Gln Val Val Gly Ser Ala Val Ala
1               5                   10

<210> 29
<211> 7
<212> PRT
<213> Mus sp.

<400> 29

Trp Ala Ser Thr Arg His Thr
1               5


<210> 30
<211> 9
<212> PRT
<213> Mus sp.

<400> 30

Gln Gln Tyr Asn Ser Tyr Pro Tyr Thr
1               5


<210> 31
<211> 5
<212> PRT
<213> Mus sp.

<400> 31

Asn Phe Gly Met His
1               5


<210> 32
<211> 17
<212> PRT
<213> Mus sp.

<400> 32

Tyr Ile Arg Ser Gly Ser Gly Thr Ile Tyr Tyr Ser Asp Thr Val Lys
1               5                   10                  15

Gly


<210> 33
<211> 11
<212> PRT
<213> Mus sp.

<400> 33

Ser Tyr Tyr Asp Phe Gly Ala Trp Phe Ala Tyr
1               5                   10


<210> 34
<211> 11
<212> PRT
<213> Mus sp.

<400> 34

Lys Ala Ser Gln Asn Val Gly Thr Asn Val Ala

```
<210>  35
<211>  7
<212>  PRT
<213>  Mus sp.

<400>  35

Ser Ala Ser Ser Arg Tyr Ser
1               5


<210>  36
<211>  9
<212>  PRT
<213>  Mus sp.

<400>  36

Gln Gln Tyr Asn Ser Tyr Pro Leu Thr
1               5


<210>  37
<211>  336
<212>  DNA
<213>  Mus sp.


<220>
<221>  CDS
<222>  (1)..(336)

<400>  37
aac att gtg ctg acc caa tct cca gct tct ttg gct gtg tct ctt ggg    48
Asn Ile Val Leu Thr Gln Ser Pro Ala Ser Leu Ala Val Ser Leu Gly
1               5                   10                  15

cag agg gcc acc ata tcc tgc aga gcc agt gaa agt gtt gag att tat    96
Gln Arg Ala Thr Ile Ser Cys Arg Ala Ser Glu Ser Val Glu Ile Tyr
                20                  25                  30

ggc aat ggt ttt atg aac tgg ttc cag cag aaa cca gga cag cca ccc    144
Gly Asn Gly Phe Met Asn Trp Phe Gln Gln Lys Pro Gly Gln Pro Pro
            35                  40                  45

aaa ctc ctc atc tat cgt gca tcc aac cta gaa tct ggg atc cct gcc    192
Lys Leu Leu Ile Tyr Arg Ala Ser Asn Leu Glu Ser Gly Ile Pro Ala
        50                  55                  60

agg ttc agt ggc agt ggg tct agg aca gag ttc acc ctc acc att gat    240
Arg Phe Ser Gly Ser Gly Ser Arg Thr Glu Phe Thr Leu Thr Ile Asp
65                  70                  75                  80

cct gtg gag gct gat gat gtt gca acc tat tac tgt caa caa att aat    288
Pro Val Glu Ala Asp Asp Val Ala Thr Tyr Tyr Cys Gln Gln Ile Asn
                85                  90                  95

gag gat cca ttc acg ttc ggc tcg ggg aca aag ttg gaa ata aaa cgg    336
Glu Asp Pro Phe Thr Phe Gly Ser Gly Thr Lys Leu Glu Ile Lys Arg
                100                 105                 110


<210>  38
<211>  112
<212>  PRT
```

<210> Mus sp.

<400> 38

| | | | | | | | | | | | | | | |
|Asn|Ile|Val|Leu|Thr|Gln|Ser|Pro|Ala|Ser|Leu|Ala|Val|Ser|Leu|Gly|
|1| | | |5| | | | |10| | | | |15| |

|Gln|Arg|Ala|Thr|Ile|Ser|Cys|Arg|Ala|Ser|Glu|Ser|Val|Glu|Ile|Tyr|
| | | |20| | | | |25| | | | |30| | |

|Gly|Asn|Gly|Phe|Met|Asn|Trp|Phe|Gln|Gln|Lys|Pro|Gly|Gln|Pro|Pro|
| | |35| | | | |40| | | | |45| | | |

|Lys|Leu|Leu|Ile|Tyr|Arg|Ala|Ser|Asn|Leu|Glu|Ser|Gly|Ile|Pro|Ala|
| |50| | | | |55| | | | |60| | | | |

|Arg|Phe|Ser|Gly|Ser|Gly|Ser|Arg|Thr|Glu|Phe|Thr|Leu|Thr|Ile|Asp|
|65| | | | |70| | | | |75| | | | |80|

|Pro|Val|Glu|Ala|Asp|Asp|Val|Ala|Thr|Tyr|Tyr|Cys|Gln|Gln|Ile|Asn|
| | | | |85| | | | |90| | | | |95| |

|Glu|Asp|Pro|Phe|Thr|Phe|Gly|Ser|Gly|Thr|Lys|Leu|Glu|Ile|Lys|Arg|
| | | |100| | | | |105| | | | |110| | |

<210> 39
<211> 336
<212> DNA
<213> Mus sp.

<220>
<221> CDS
<222> (1)..(336)

<400> 39

```
gac att gta ctg acc caa tct cca gct tct ttg gct gtg tct cta ggg      48
Asp Ile Val Leu Thr Gln Ser Pro Ala Ser Leu Ala Val Ser Leu Gly
1               5                   10                  15

cag agg gcc acc ata tcc tgc aga gcc agt gag agt gtt gct att tat      96
Gln Arg Ala Thr Ile Ser Cys Arg Ala Ser Glu Ser Val Ala Ile Tyr
                20                  25                  30

ggc aat agt ttt ctg aaa tgg ttc cag cag aaa ccg gga cag cca ccc     144
Gly Asn Ser Phe Leu Lys Trp Phe Gln Gln Lys Pro Gly Gln Pro Pro
            35                  40                  45

aaa ctc ctc atc tat cgt gca tcc aac cta gaa tct ggg atc cct gcc     192
Lys Leu Leu Ile Tyr Arg Ala Ser Asn Leu Glu Ser Gly Ile Pro Ala
        50                  55                  60

agg ttc agt ggc agt ggg tct ggg aca gac ttc acc ctc acc att aat     240
Arg Phe Ser Gly Ser Gly Ser Gly Thr Asp Phe Thr Leu Thr Ile Asn
65                  70                  75                  80

cct gtg gag gct gat gat gtt gca acc tat tac tgt cag caa att aat     288
Pro Val Glu Ala Asp Asp Val Ala Thr Tyr Tyr Cys Gln Gln Ile Asn
                85                  90                  95

gag gat ccg tac acg ttc gga ggg ggg acc aag ctg gaa ata aaa cgg     336
```

```
Glu Asp Pro Tyr Thr Phe Gly Gly Gly Thr Lys Leu Glu Ile Lys Arg
            100                 105                 110
```

```
<210>   40
<211>   112
<212>   PRT
<213>   Mus sp.
```

```
<400>   40
```

```
Asp Ile Val Leu Thr Gln Ser Pro Ala Ser Leu Ala Val Ser Leu Gly
1                 5                 10                  15
```

```
Gln Arg Ala Thr Ile Ser Cys Arg Ala Ser Glu Ser Val Ala Ile Tyr
            20                  25                  30
```

```
Gly Asn Ser Phe Leu Lys Trp Phe Gln Gln Lys Pro Gly Gln Pro Pro
        35                  40                  45
```

```
Lys Leu Leu Ile Tyr Arg Ala Ser Asn Leu Glu Ser Gly Ile Pro Ala
    50                  55                  60
```

```
Arg Phe Ser Gly Ser Gly Ser Gly Thr Asp Phe Thr Leu Thr Ile Asn
65                  70                  75                  80
```

```
Pro Val Glu Ala Asp Asp Val Ala Thr Tyr Tyr Cys Gln Gln Ile Asn
                85                  90                  95
```

```
Glu Asp Pro Tyr Thr Phe Gly Gly Gly Thr Lys Leu Glu Ile Lys Arg
            100                 105                 110
```

```
<210>   41
<211>   324
<212>   DNA
<213>   Mus sp.
```

```
<220>
<221>   CDS
<222>   (1)..(324)
```

```
<400>   41
gac att gtg atg gcc cag tct cac aaa ttc atg tcc aca tca gtt gga      48
Asp Ile Val Met Ala Gln Ser His Lys Phe Met Ser Thr Ser Val Gly
1                 5                 10                  15

gac agg gtc agc atc acc tgc aag gcc agt cag gat gtg agt act gtt      96
Asp Arg Val Ser Ile Thr Cys Lys Ala Ser Gln Asp Val Ser Thr Val
            20                  25                  30

gtg gcc tgg tat caa cag aaa cca gga caa tct cct aaa cga ctg att     144
Val Ala Trp Tyr Gln Gln Lys Pro Gly Gln Ser Pro Lys Arg Leu Ile
        35                  40                  45

tac tcg gca tcc tat cgg tat att gga gtc cct gat cgc ttc act ggc     192
Tyr Ser Ala Ser Tyr Arg Tyr Ile Gly Val Pro Asp Arg Phe Thr Gly
    50                  55                  60

agt gga tct ggg acg gat ttc act ttc acc atc agc agt gtg cag gct     240
Ser Gly Ser Gly Thr Asp Phe Thr Phe Thr Ile Ser Ser Val Gln Ala
```

```
                65                    70                    75                    80
                gaa gac ctg gca gtt tat tac tgt cag caa cat tat agt cct ccg tac         288
                Glu Asp Leu Ala Val Tyr Tyr Cys Gln Gln His Tyr Ser Pro Pro Tyr
                                85                    90                    95

                acg ttc gga ggg ggg acc aag ctg gaa ata aaa cgg                         324
                Thr Phe Gly Gly Gly Thr Lys Leu Glu Ile Lys Arg
                                100                   105


                <210>   42
                <211>   108
                <212>   PRT
                <213>   Mus sp.

                <400>   42

                Asp Ile Val Met Ala Gln Ser His Lys Phe Met Ser Thr Ser Val Gly
                1                   5                   10                    15


                Asp Arg Val Ser Ile Thr Cys Lys Ala Ser Gln Asp Val Ser Thr Val
                                20                    25                    30


                Val Ala Trp Tyr Gln Gln Lys Pro Gly Gln Ser Pro Lys Arg Leu Ile
                                35                    40                    45


                Tyr Ser Ala Ser Tyr Arg Tyr Ile Gly Val Pro Asp Arg Phe Thr Gly
                                50                    55                    60


                Ser Gly Ser Gly Thr Asp Phe Thr Phe Thr Ile Ser Ser Val Gln Ala
                65                    70                    75                    80


                Glu Asp Leu Ala Val Tyr Tyr Cys Gln Gln His Tyr Ser Pro Pro Tyr
                                85                    90                    95


                Thr Phe Gly Gly Gly Thr Lys Leu Glu Ile Lys Arg
                                100                   105


                <210>   43
                <211>   324
                <212>   DNA
                <213>   Mus sp.


                <220>
                <221>   CDS
                <222>   (1)..(324)

                <400>   43
                gac att gtg atg acc cag tct cac aaa ttc ttg tcc aca tca gtt gga         48
                Asp Ile Val Met Thr Gln Ser His Lys Phe Leu Ser Thr Ser Val Gly
                1                   5                   10                    15

                gac agg gtc agt atc acc tgc aag gcc agt cag gat gtg gtt act gct         96
                Asp Arg Val Ser Ile Thr Cys Lys Ala Ser Gln Asp Val Val Thr Ala
                                20                    25                    30

                gtt gcc tgg ttt caa cag aaa cca gga caa tct cca aaa cta ctg att        144
                Val Ala Trp Phe Gln Gln Lys Pro Gly Gln Ser Pro Lys Leu Leu Ile
                                35                    40                    45
```

17

```
tat tcg gca tcc cac cgg tac act gga gtc cct gat cgc ttc act ggc        192
Tyr Ser Ala Ser His Arg Tyr Thr Gly Val Pro Asp Arg Phe Thr Gly
    50                  55                  60

agt gga tct ggg aca gat ttc act ttc acc atc atc agt gtg cag gct        240
Ser Gly Ser Gly Thr Asp Phe Thr Phe Thr Ile Ile Ser Val Gln Ala
65                  70                  75                  80

gaa gac ctg gca gtt tat tac tgt caa caa cat tat act act ccc acg        288
Glu Asp Leu Ala Val Tyr Tyr Cys Gln Gln His Tyr Thr Thr Pro Thr
                85                  90                  95

acg ttc ggt gga ggc acc aag ctg gac ttc aga cgg                        324
Thr Phe Gly Gly Gly Thr Lys Leu Asp Phe Arg Arg
            100                 105
```

<210> 44
<211> 108
<212> PRT
<213> Mus sp.

<400> 44

```
Asp Ile Val Met Thr Gln Ser His Lys Phe Leu Ser Thr Ser Val Gly
1               5                   10                  15

Asp Arg Val Ser Ile Thr Cys Lys Ala Ser Gln Asp Val Val Thr Ala
            20                  25                  30

Val Ala Trp Phe Gln Gln Lys Pro Gly Gln Ser Pro Lys Leu Leu Ile
        35                  40                  45

Tyr Ser Ala Ser His Arg Tyr Thr Gly Val Pro Asp Arg Phe Thr Gly
    50                  55                  60

Ser Gly Ser Gly Thr Asp Phe Thr Phe Thr Ile Ile Ser Val Gln Ala
65                  70                  75                  80

Glu Asp Leu Ala Val Tyr Tyr Cys Gln Gln His Tyr Thr Thr Pro Thr
                85                  90                  95

Thr Phe Gly Gly Gly Thr Lys Leu Asp Phe Arg Arg
            100                 105
```

<210> 45
<211> 324
<212> DNA
<213> Mus sp.

<220>
<221> CDS
<222> (1)..(324)

<400> 45
```
gac act gtg atg acc cag tct cac aaa ttc ata tcc aca tca gtt gga        48
Asp Thr Val Met Thr Gln Ser His Lys Phe Ile Ser Thr Ser Val Gly
1               5                   10                  15
```

```
gac agg gtc agc atc acc tgc aag gcc agt cag gtt gtg ggt agt gct        96
Asp Arg Val Ser Ile Thr Cys Lys Ala Ser Gln Val Val Gly Ser Ala
            20              25              30

gta gcc tgg tat caa cag aaa cca ggg caa tct cct aaa cta ctg att       144
Val Ala Trp Tyr Gln Gln Lys Pro Gly Gln Ser Pro Lys Leu Leu Ile
            35              40              45

tac tgg gca tcc acc cgg cac act gga gtc cct gat cgc ttc aca ggc       192
Tyr Trp Ala Ser Thr Arg His Thr Gly Val Pro Asp Arg Phe Thr Gly
            50              55              60

agt gga tct ggg aca gat ttc act ctc acc att agc aat gtg cag tct       240
Ser Gly Ser Gly Thr Asp Phe Thr Leu Thr Ile Ser Asn Val Gln Ser
65              70              75              80

gaa gac ttg gca gat tat ttc tgt cag caa tat aac agc tat ccg tac       288
Glu Asp Leu Ala Asp Tyr Phe Cys Gln Gln Tyr Asn Ser Tyr Pro Tyr
            85              90              95

acg ttc gga ggg ggg acc aag ctg gaa ata aaa cgg                       324
Thr Phe Gly Gly Gly Thr Lys Leu Glu Ile Lys Arg
            100             105
```

<210> 46
<211> 108
<212> PRT
<213> Mus sp.

<400> 46

```
Asp Thr Val Met Thr Gln Ser His Lys Phe Ile Ser Thr Ser Val Gly
1               5               10              15

Asp Arg Val Ser Ile Thr Cys Lys Ala Ser Gln Val Val Gly Ser Ala
            20              25              30

Val Ala Trp Tyr Gln Gln Lys Pro Gly Gln Ser Pro Lys Leu Leu Ile
            35              40              45

Tyr Trp Ala Ser Thr Arg His Thr Gly Val Pro Asp Arg Phe Thr Gly
            50              55              60

Ser Gly Ser Gly Thr Asp Phe Thr Leu Thr Ile Ser Asn Val Gln Ser
65              70              75              80

Glu Asp Leu Ala Asp Tyr Phe Cys Gln Gln Tyr Asn Ser Tyr Pro Tyr
            85              90              95

Thr Phe Gly Gly Gly Thr Lys Leu Glu Ile Lys Arg
            100             105
```

<210> 47
<211> 324
<212> DNA
<213> Mus sp.

<220>
<221> CDS

<222> (1)..(324)

<400> 47

```
gac att gtg atg acc cag tct caa aaa ttc atg tcc aca tca gta gga        48
Asp Ile Val Met Thr Gln Ser Gln Lys Phe Met Ser Thr Ser Val Gly
1               5                   10                  15

gac agg gtc agc gtc acc tgc aag gcc agt cag aat gtg ggt act aat        96
Asp Arg Val Ser Val Thr Cys Lys Ala Ser Gln Asn Val Gly Thr Asn
            20                  25                  30

gtt gcc tgg tat caa cac aaa cca gga caa tcc cct aaa ata atg att       144
Val Ala Trp Tyr Gln His Lys Pro Gly Gln Ser Pro Lys Ile Met Ile
        35                  40                  45

tat tcg gcg tcc tcc cgg tac agt gga gtc cct gat cgc ttc aca ggc       192
Tyr Ser Ala Ser Ser Arg Tyr Ser Gly Val Pro Asp Arg Phe Thr Gly
    50                  55                  60

agt gga tct ggg aca ctt ttc act ctc acc atc aac aat gtg cag tct       240
Ser Gly Ser Gly Thr Leu Phe Thr Leu Thr Ile Asn Asn Val Gln Ser
65                  70                  75                  80

gaa gac ttg gca gag tat ttc tgt cag caa tat aac agc tat cct ctc       288
Glu Asp Leu Ala Glu Tyr Phe Cys Gln Gln Tyr Asn Ser Tyr Pro Leu
                85                  90                  95

acg ttc ggc tcg ggg aca aag ttg gaa ata aaa cgg                        324
Thr Phe Gly Ser Gly Thr Lys Leu Glu Ile Lys Arg
                100                 105
```

<210> 48
<211> 108
<212> PRT
<213> Mus sp.

<400> 48

```
Asp Ile Val Met Thr Gln Ser Gln Lys Phe Met Ser Thr Ser Val Gly
1               5                   10                  15

Asp Arg Val Ser Val Thr Cys Lys Ala Ser Gln Asn Val Gly Thr Asn
            20                  25                  30

Val Ala Trp Tyr Gln His Lys Pro Gly Gln Ser Pro Lys Ile Met Ile
        35                  40                  45

Tyr Ser Ala Ser Ser Arg Tyr Ser Gly Val Pro Asp Arg Phe Thr Gly
    50                  55                  60

Ser Gly Ser Gly Thr Leu Phe Thr Leu Thr Ile Asn Asn Val Gln Ser
65                  70                  75                  80

Glu Asp Leu Ala Glu Tyr Phe Cys Gln Gln Tyr Asn Ser Tyr Pro Leu
                85                  90                  95

Thr Phe Gly Ser Gly Thr Lys Leu Glu Ile Lys Arg
                100                 105
```

<210> 49

```
<211>   342
<212>   DNA
<213>   Mus sp.


<220>
<221>   CDS
<222>   (1)..(342)

<400>   49
cag atc cag ttg gtg cag tct gga cct gag ctg aag aag cct gga gag    48
Gln Ile Gln Leu Val Gln Ser Gly Pro Glu Leu Lys Lys Pro Gly Glu
1               5                   10                  15

aca gtc aag atc tcc tgc aag gct tct ggg tat acc ctc aca agc tac    96
Thr Val Lys Ile Ser Cys Lys Ala Ser Gly Tyr Thr Leu Thr Ser Tyr
            20                  25                  30

gga atg aac tgg gtg aag cag gct cca gga aag ggt tta aag tgg atg   144
Gly Met Asn Trp Val Lys Gln Ala Pro Gly Lys Gly Leu Lys Trp Met
        35                  40                  45

ggc tgg ata aac acc tac act gga gaa cca aca tat gct gat gac ttt   192
Gly Trp Ile Asn Thr Tyr Thr Gly Glu Pro Thr Tyr Ala Asp Asp Phe
    50                  55                  60

aag gga cgt ttt gcc ttc tct ttg gaa acc tct gcc agc act gcc ttt   240
Lys Gly Arg Phe Ala Phe Ser Leu Glu Thr Ser Ala Ser Thr Ala Phe
65                  70                  75                  80

ttg cag atc aac aac ctc aaa aat gag gac acg gct aca tat ttc tgt   288
Leu Gln Ile Asn Asn Leu Lys Asn Glu Asp Thr Ala Thr Tyr Phe Cys
                85                  90                  95

gta aga cgc ggg ttt gct tac tgg ggc caa ggg act ctg gtc act gtc   336
Val Arg Arg Gly Phe Ala Tyr Trp Gly Gln Gly Thr Leu Val Thr Val
            100                 105                 110

tct gca                                                            342
Ser Ala



<210>   50
<211>   114
<212>   PRT
<213>   Mus sp.

<400>   50

Gln Ile Gln Leu Val Gln Ser Gly Pro Glu Leu Lys Lys Pro Gly Glu
1               5                   10                  15


Thr Val Lys Ile Ser Cys Lys Ala Ser Gly Tyr Thr Leu Thr Ser Tyr
            20                  25                  30


Gly Met Asn Trp Val Lys Gln Ala Pro Gly Lys Gly Leu Lys Trp Met
        35                  40                  45


Gly Trp Ile Asn Thr Tyr Thr Gly Glu Pro Thr Tyr Ala Asp Asp Phe
    50                  55                  60


Lys Gly Arg Phe Ala Phe Ser Leu Glu Thr Ser Ala Ser Thr Ala Phe
65                  70                  75                  80
```

```
Leu Gln Ile Asn Asn Leu Lys Asn Glu Asp Thr Ala Thr Tyr Phe Cys
                 85                  90                  95


Val Arg Arg Gly Phe Ala Tyr Trp Gly Gln Gly Thr Leu Val Thr Val
             100                 105                 110


Ser Ala



<210>   51
<211>   342
<212>   DNA
<213>   Mus sp.


<220>
<221>   CDS
<222>   (1)..(342)

<400>   51
cag atc cag ttg gtg cag tct gga cct gag ctg aag aag cct gga gag    48
Gln Ile Gln Leu Val Gln Ser Gly Pro Glu Leu Lys Lys Pro Gly Glu
1               5                   10                  15

aca gtc aag atc tcc tgc aag gct tct ggg tat acc ttc aca aac tct    96
Thr Val Lys Ile Ser Cys Lys Ala Ser Gly Tyr Thr Phe Thr Asn Ser
            20                  25                  30

gga atg aac tgg gtg aag cag gct cca gga aag ggt tta aag tgg atg   144
Gly Met Asn Trp Val Lys Gln Ala Pro Gly Lys Gly Leu Lys Trp Met
        35                  40                  45

ggc tgg ata aac acc tac act gga gag ccg aca tat gct gat gac ttc   192
Gly Trp Ile Asn Thr Tyr Thr Gly Glu Pro Thr Tyr Ala Asp Asp Phe
    50                  55                  60

aag gga cgg ttt gcc ttc tct ttg gaa acc tct gcc agc tct gcc tat   240
Lys Gly Arg Phe Ala Phe Ser Leu Glu Thr Ser Ala Ser Ser Ala Tyr
65                  70                  75                  80

ttg cag atc agt aac ctc aaa aat gag gac acg gct aca tat ttc tgt   288
Leu Gln Ile Ser Asn Leu Lys Asn Glu Asp Thr Ala Thr Tyr Phe Cys
                85                  90                  95

gca aga agg ggt ttt gtt tac tgg ggc caa ggg act ctg gta act gtc   336
Ala Arg Arg Gly Phe Val Tyr Trp Gly Gln Gly Thr Leu Val Thr Val
            100                 105                 110

tct gca                                                             342
Ser Ala



<210>   52
<211>   114
<212>   PRT
<213>   Mus sp.

<400>   52

Gln Ile Gln Leu Val Gln Ser Gly Pro Glu Leu Lys Lys Pro Gly Glu
1               5                   10                  15
```

```
Thr Val Lys Ile Ser Cys Lys Ala Ser Gly Tyr Thr Phe Thr Asn Ser
              20                25                30

Gly Met Asn Trp Val Lys Gln Ala Pro Gly Lys Gly Leu Lys Trp Met
              35                40                45

Gly Trp Ile Asn Thr Tyr Thr Gly Glu Pro Thr Tyr Ala Asp Asp Phe
              50                55                60

Lys Gly Arg Phe Ala Phe Ser Leu Glu Thr Ser Ala Ser Ser Ala Tyr
65                70                75                80

Leu Gln Ile Ser Asn Leu Lys Asn Glu Asp Thr Ala Thr Tyr Phe Cys
                  85                90                95

Ala Arg Arg Gly Phe Val Tyr Trp Gly Gln Gly Thr Leu Val Thr Val
              100               105               110

Ser Ala
```

```
<210>   53
<211>   360
<212>   DNA
<213>   Mus sp.


<220>
<221>   CDS
<222>   (1)..(360)

<400>   53
cag gtt cag ctc cag cag tct ggg gct gag ctg gca aga cct ggg act     48
Gln Val Gln Leu Gln Gln Ser Gly Ala Glu Leu Ala Arg Pro Gly Thr
1               5                 10                15

tca gtg aag ttg tcc tgt aag gct tct ggc tac acc ttt act gac tac     96
Ser Val Lys Leu Ser Cys Lys Ala Ser Gly Tyr Thr Phe Thr Asp Tyr
              20                25                30

tgg atg cag tgg gta aaa cag agg cct gga cag ggt ctg gag tgg att    144
Trp Met Gln Trp Val Lys Gln Arg Pro Gly Gln Gly Leu Glu Trp Ile
              35                40                45

ggg act att tat cct gga gat ggt gat act ggg tac gct cag aag ttc    192
Gly Thr Ile Tyr Pro Gly Asp Gly Asp Thr Gly Tyr Ala Gln Lys Phe
              50                55                60

aag ggc aag gcc aca ttg act gcg gat aaa tcc tcc aaa aca gtc tac    240
Lys Gly Lys Ala Thr Leu Thr Ala Asp Lys Ser Ser Lys Thr Val Tyr
65                70                75                80

atg cac ctc agc agt ttg gct tct gag gac tct gcg gtc tat tac tgt    288
Met His Leu Ser Ser Leu Ala Ser Glu Asp Ser Ala Val Tyr Tyr Cys
                  85                90                95

gca aga ggg gat tac tac ggt agt aat tct ttg gac tat tgg ggt caa    336
Ala Arg Gly Asp Tyr Tyr Gly Ser Asn Ser Leu Asp Tyr Trp Gly Gln
              100               105               110
```

```
gga acc tca gtc acc gtc tcc tca                                360
Gly Thr Ser Val Thr Val Ser Ser
        115             120


<210>  54
<211>  120
<212>  PRT
<213>  Mus sp.

<400>  54

Gln Val Gln Leu Gln Gln Ser Gly Ala Glu Leu Ala Arg Pro Gly Thr
1               5               10              15

Ser Val Lys Leu Ser Cys Lys Ala Ser Gly Tyr Thr Phe Thr Asp Tyr
            20              25              30

Trp Met Gln Trp Val Lys Gln Arg Pro Gly Gln Gly Leu Glu Trp Ile
        35              40              45

Gly Thr Ile Tyr Pro Gly Asp Gly Asp Thr Gly Tyr Ala Gln Lys Phe
    50              55              60

Lys Gly Lys Ala Thr Leu Thr Ala Asp Lys Ser Ser Lys Thr Val Tyr
65              70              75              80

Met His Leu Ser Ser Leu Ala Ser Glu Asp Ser Ala Val Tyr Tyr Cys
            85              90              95

Ala Arg Gly Asp Tyr Tyr Gly Ser Asn Ser Leu Asp Tyr Trp Gly Gln
            100             105             110

Gly Thr Ser Val Thr Val Ser Ser
        115             120


<210>  55
<211>  357
<212>  DNA
<213>  Mus sp.


<220>
<221>  CDS
<222>  (1)..(357)

<400>  55
cag gtc cag tta cag caa tct gga cct gaa ctg gtg agg cct ggg gcc   48
Gln Val Gln Leu Gln Gln Ser Gly Pro Glu Leu Val Arg Pro Gly Ala
1               5               10              15

tca gtg aag att tcc tgc aaa act tct ggc tac gca ttc agt ggc tcc   96
Ser Val Lys Ile Ser Cys Lys Thr Ser Gly Tyr Ala Phe Ser Gly Ser
            20              25              30

tgg atg aac tgg gtg aag cag agg cct gga cag ggt cta gag tgg att  144
Trp Met Asn Trp Val Lys Gln Arg Pro Gly Gln Gly Leu Glu Trp Ile
        35              40              45

gga cgg att tat ccg gga gat gga gat atc att tac aat ggg aat ttc  192
```

```
Gly Arg Ile Tyr Pro Gly Asp Gly Asp Ile Ile Tyr Asn Gly Asn Phe
    50              55              60

agg gac aag gtc aca ctg tct gca gac aaa tcc tcc aac aca gcc tac    240
Arg Asp Lys Val Thr Leu Ser Ala Asp Lys Ser Ser Asn Thr Ala Tyr
65              70              75              80

atg cag ctc agc agc ctg acc tct gtg gac tct gcg gtc tat ttt tgt    288
Met Gln Leu Ser Ser Leu Thr Ser Val Asp Ser Ala Val Tyr Phe Cys
            85              90              95

tcg aga tgg ggg aca ttt acg ccg agt ttt gac tat tgg ggc caa ggc    336
Ser Arg Trp Gly Thr Phe Thr Pro Ser Phe Asp Tyr Trp Gly Gln Gly
            100             105             110

acc act ctc aca gtc tcc tca                                        357
Thr Thr Leu Thr Val Ser Ser
            115
```

<210> 56
<211> 119
<212> PRT
<213> Mus sp.

<400> 56

```
Gln Val Gln Leu Gln Gln Ser Gly Pro Glu Leu Val Arg Pro Gly Ala
1               5               10              15

Ser Val Lys Ile Ser Cys Lys Thr Ser Gly Tyr Ala Phe Ser Gly Ser
            20              25              30

Trp Met Asn Trp Val Lys Gln Arg Pro Gly Gln Gly Leu Glu Trp Ile
            35              40              45

Gly Arg Ile Tyr Pro Gly Asp Gly Asp Ile Ile Tyr Asn Gly Asn Phe
    50              55              60

Arg Asp Lys Val Thr Leu Ser Ala Asp Lys Ser Ser Asn Thr Ala Tyr
65              70              75              80

Met Gln Leu Ser Ser Leu Thr Ser Val Asp Ser Ala Val Tyr Phe Cys
            85              90              95

Ser Arg Trp Gly Thr Phe Thr Pro Ser Phe Asp Tyr Trp Gly Gln Gly
            100             105             110

Thr Thr Leu Thr Val Ser Ser
            115
```

<210> 57
<211> 351
<212> DNA
<213> Mus sp.

<220>
<221> CDS
<222> (1)..(351)

<400> 57

```
gac gtg aag ctg gtg gag tct ggg gga ggc tta gtg aag cct gga ggg          48
Asp Val Lys Leu Val Glu Ser Gly Gly Gly Leu Val Lys Pro Gly Gly
1               5                   10                  15

tcc ctg aaa ctc tcc tgt gaa gcc tct gga ttc act ttc agt agc tat          96
Ser Leu Lys Leu Ser Cys Glu Ala Ser Gly Phe Thr Phe Ser Ser Tyr
                20                  25                  30

acc ctg tct tgg gtt cgc cag act ccg gag acg agg ctg gag tgg gtc          144
Thr Leu Ser Trp Val Arg Gln Thr Pro Glu Thr Arg Leu Glu Trp Val
            35                  40                  45

gca acc att agt att ggt ggt cgc tac acc tat tat cca gac agt gtg          192
Ala Thr Ile Ser Ile Gly Gly Arg Tyr Thr Tyr Tyr Pro Asp Ser Val
        50                  55                  60

gag ggc cga ttc acc atc tcc aga gac aat gcc aag aac acc ctg tac          240
Glu Gly Arg Phe Thr Ile Ser Arg Asp Asn Ala Lys Asn Thr Leu Tyr
65                  70                  75                  80

ctg caa atg aac agt ctg aag tct gag gac aca gcc atg tat tac tgt          288
Leu Gln Met Asn Ser Leu Lys Ser Glu Asp Thr Ala Met Tyr Tyr Cys
                85                  90                  95

aca aga gat ttt aat ggt tac tct gac ttc tgg ggc caa ggc acc act          336
Thr Arg Asp Phe Asn Gly Tyr Ser Asp Phe Trp Gly Gln Gly Thr Thr
                100                 105                 110

ctc aca gtc tcc tca                                                       351
Leu Thr Val Ser Ser
                115
```

<210> 58
<211> 117
<212> PRT
<213> Mus sp.

<400> 58

```
Asp Val Lys Leu Val Glu Ser Gly Gly Gly Leu Val Lys Pro Gly Gly
1               5                   10                  15

Ser Leu Lys Leu Ser Cys Glu Ala Ser Gly Phe Thr Phe Ser Ser Tyr
                20                  25                  30

Thr Leu Ser Trp Val Arg Gln Thr Pro Glu Thr Arg Leu Glu Trp Val
            35                  40                  45

Ala Thr Ile Ser Ile Gly Gly Arg Tyr Thr Tyr Tyr Pro Asp Ser Val
        50                  55                  60

Glu Gly Arg Phe Thr Ile Ser Arg Asp Asn Ala Lys Asn Thr Leu Tyr
65                  70                  75                  80

Leu Gln Met Asn Ser Leu Lys Ser Glu Asp Thr Ala Met Tyr Tyr Cys
                85                  90                  95

Thr Arg Asp Phe Asn Gly Tyr Ser Asp Phe Trp Gly Gln Gly Thr Thr
                100                 105                 110
```

```
Leu Thr Val Ser Ser
        115


<210>  59
<211>  360
<212>  DNA
<213>  Mus sp.


<220>
<221>  CDS
<222>  (1)..(360)

<400>  59
aat gta cag ctg gta gag tct ggg gga ggc tta gtg cag cct gga ggg      48
Asn Val Gln Leu Val Glu Ser Gly Gly Gly Leu Val Gln Pro Gly Gly
1               5                   10                  15

tcc cgg aaa ctc tcc tgt gca gcc tct gga ttc act ttc agt aac ttt      96
Ser Arg Lys Leu Ser Cys Ala Ala Ser Gly Phe Thr Phe Ser Asn Phe
            20                  25                  30

gga atg cac tgg gtt cgt cag gct cca gag aag ggt ctg gag tgg gtc     144
Gly Met His Trp Val Arg Gln Ala Pro Glu Lys Gly Leu Glu Trp Val
        35                  40                  45

gca tac att cgt agt ggc agt ggt acc atc tac tat tca gac aca gtg     192
Ala Tyr Ile Arg Ser Gly Ser Gly Thr Ile Tyr Tyr Ser Asp Thr Val
    50                  55                  60

aag ggc cga ttc acc atc tcc aga gac aat ccc aag aac acc ctg ttc     240
Lys Gly Arg Phe Thr Ile Ser Arg Asp Asn Pro Lys Asn Thr Leu Phe
65                  70                  75                  80

ctg caa atg acc agt cta agg tct gag gac acg gcc atg tat tac tgt     288
Leu Gln Met Thr Ser Leu Arg Ser Glu Asp Thr Ala Met Tyr Tyr Cys
                85                  90                  95

gca aga tcc tac tat gat ttc ggg gcc tgg ttt gct tac tgg ggc caa     336
Ala Arg Ser Tyr Tyr Asp Phe Gly Ala Trp Phe Ala Tyr Trp Gly Gln
            100                 105                 110

ggg act ctg gtc act gtc tct gca                                      360
Gly Thr Leu Val Thr Val Ser Ala
        115                 120


<210>  60
<211>  120
<212>  PRT
<213>  Mus sp.

<400>  60

Asn Val Gln Leu Val Glu Ser Gly Gly Gly Leu Val Gln Pro Gly Gly
1               5                   10                  15


Ser Arg Lys Leu Ser Cys Ala Ala Ser Gly Phe Thr Phe Ser Asn Phe
            20                  25                  30


Gly Met His Trp Val Arg Gln Ala Pro Glu Lys Gly Leu Glu Trp Val
        35                  40                  45
```

```
Ala Tyr Ile Arg Ser Gly Ser Gly Thr Ile Tyr Tyr Ser Asp Thr Val
    50              55              60
```

```
Lys Gly Arg Phe Thr Ile Ser Arg Asp Asn Pro Lys Asn Thr Leu Phe
65              70              75              80
```

```
Leu Gln Met Thr Ser Leu Arg Ser Glu Asp Thr Ala Met Tyr Tyr Cys
            85              90              95
```

```
Ala Arg Ser Tyr Tyr Asp Phe Gly Ala Trp Phe Ala Tyr Trp Gly Gln
            100             105             110
```

```
Gly Thr Leu Val Thr Val Ser Ala
        115             120
```

```
<210>   61
<211>   324
<212>   DNA
<213>   Homo sapiens
```

```
<220>
<221>   CDS
<222>   (1)..(324)
```

```
<400>   61
gat atc gta atg acc cag tcc cac ctg agt atg agt acc tcc ctg gga      48
Asp Ile Val Met Thr Gln Ser His Leu Ser Met Ser Thr Ser Leu Gly
1               5               10              15
```

```
gat cct gtg tca atc act tgc aag gcc tca cag gat gtg agc acc gtc      96
Asp Pro Val Ser Ile Thr Cys Lys Ala Ser Gln Asp Val Ser Thr Val
            20              25              30
```

```
gtt gct tgg tat cag cag aag ccc ggg caa tca ccc aga cgt ctc atc     144
Val Ala Trp Tyr Gln Gln Lys Pro Gly Gln Ser Pro Arg Arg Leu Ile
        35              40              45
```

```
tac tca gca tca tac cgt tac atc ggg gtg cct gac cga ttt act ggc     192
Tyr Ser Ala Ser Tyr Arg Tyr Ile Gly Val Pro Asp Arg Phe Thr Gly
    50              55              60
```

```
tct ggc gct ggc aca gat ttc acc ttt aca att agt tcc gtc cag gcc     240
Ser Gly Ala Gly Thr Asp Phe Thr Phe Thr Ile Ser Ser Val Gln Ala
65              70              75              80
```

```
gaa gac ctg gcc gtg tac tac tgc cag cag cac tac agt ccc cca tac     288
Glu Asp Leu Ala Val Tyr Tyr Cys Gln Gln His Tyr Ser Pro Pro Tyr
            85              90              95
```

```
act ttc ggg gga ggg act aag ctc gaa atc aaa cgt                      324
Thr Phe Gly Gly Gly Thr Lys Leu Glu Ile Lys Arg
            100             105
```

```
<210>   62
<211>   108
<212>   PRT
<213>   Homo sapiens
```

```
<400>   62
```

```
Asp Ile Val Met Thr Gln Ser His Leu Ser Met Ser Thr Ser Leu Gly
1               5                   10                  15

Asp Pro Val Ser Ile Thr Cys Lys Ala Ser Gln Asp Val Ser Thr Val
            20                  25                  30

Val Ala Trp Tyr Gln Gln Lys Pro Gly Gln Ser Pro Arg Arg Leu Ile
        35                  40                  45

Tyr Ser Ala Ser Tyr Arg Tyr Ile Gly Val Pro Asp Arg Phe Thr Gly
    50                  55                  60

Ser Gly Ala Gly Thr Asp Phe Thr Phe Thr Ile Ser Ser Val Gln Ala
65                  70                  75                  80

Glu Asp Leu Ala Val Tyr Tyr Cys Gln Gln His Tyr Ser Pro Pro Tyr
            85                  90                  95

Thr Phe Gly Gly Gly Thr Lys Leu Glu Ile Lys Arg
            100                 105
```

```
<210>   63
<211>   324
<212>   DNA
<213>   Homo sapiens


<220>
<221>   CDS
<222>   (1)..(324)

<400>   63
gac att gtt atg gct caa agc cat ctg tct atg agc aca tct ctg gga        48
Asp Ile Val Met Ala Gln Ser His Leu Ser Met Ser Thr Ser Leu Gly
1               5                   10                  15

gat cct gtg tcc atc act tgc aaa gcc agt caa gac gtg tct aca gtt        96
Asp Pro Val Ser Ile Thr Cys Lys Ala Ser Gln Asp Val Ser Thr Val
            20                  25                  30

gtt gca tgg tat caa cag aag cca ggc cag tca ccc aga cgg ctc att       144
Val Ala Trp Tyr Gln Gln Lys Pro Gly Gln Ser Pro Arg Arg Leu Ile
        35                  40                  45

tac tca gct tct tac cga tac atc ggg gtc cct gac aga ttt aca ggt       192
Tyr Ser Ala Ser Tyr Arg Tyr Ile Gly Val Pro Asp Arg Phe Thr Gly
    50                  55                  60

agt ggg gcc ggt act gac ttc act ttt act atc tca tcc gta caa gcc       240
Ser Gly Ala Gly Thr Asp Phe Thr Phe Thr Ile Ser Ser Val Gln Ala
65                  70                  75                  80

gaa gac ctg gca gta tat tac tgc cag caa cat tat tcc cca ccc tac       288
Glu Asp Leu Ala Val Tyr Tyr Cys Gln Gln His Tyr Ser Pro Pro Tyr
            85                  90                  95

aca ttc ggc ggg ggt act aag ctg gaa att aaa cgt                       324
Thr Phe Gly Gly Gly Thr Lys Leu Glu Ile Lys Arg
            100                 105
```

```
<210>    64
<211>    108
<212>    PRT
<213>    Homo sapiens

<400>    64

Asp Ile Val Met Ala Gln Ser His Leu Ser Met Ser Thr Ser Leu Gly
1                   5                   10                  15

Asp Pro Val Ser Ile Thr Cys Lys Ala Ser Gln Asp Val Ser Thr Val
                20                  25                  30

Val Ala Trp Tyr Gln Gln Lys Pro Gly Gln Ser Pro Arg Arg Leu Ile
            35                  40                  45

Tyr Ser Ala Ser Tyr Arg Tyr Ile Gly Val Pro Asp Arg Phe Thr Gly
        50                  55                  60

Ser Gly Ala Gly Thr Asp Phe Thr Phe Thr Ile Ser Ser Val Gln Ala
65                  70                  75                  80

Glu Asp Leu Ala Val Tyr Tyr Cys Gln Gln His Tyr Ser Pro Pro Tyr
                85                  90                  95

Thr Phe Gly Gly Gly Thr Lys Leu Glu Ile Lys Arg
            100                 105


<210>    65
<211>    360
<212>    DNA
<213>    Homo sapiens


<220>
<221>    CDS
<222>    (1)..(360)

<400>    65
cag gta cag ctc gtt cag tcc ggc gcc gag gta gct aag cct ggt act      48
Gln Val Gln Leu Val Gln Ser Gly Ala Glu Val Ala Lys Pro Gly Thr
1                   5                   10                  15

tcc gta aaa ttg tcc tgt aag gct tcc ggg tac aca ttt aca gac tac      96
Ser Val Lys Leu Ser Cys Lys Ala Ser Gly Tyr Thr Phe Thr Asp Tyr
                20                  25                  30

tgg atg cag tgg gta aaa cag cgg cca ggt cag ggc ctg gag tgg att     144
Trp Met Gln Trp Val Lys Gln Arg Pro Gly Gln Gly Leu Glu Trp Ile
            35                  40                  45

gga aca ata tat ccc ggc gac ggc gac aca ggc tat gcc cag aag ttt     192
Gly Thr Ile Tyr Pro Gly Asp Gly Asp Thr Gly Tyr Ala Gln Lys Phe
        50                  55                  60

caa ggc aag gca acc ctt act gct gat aaa tct tcc aag act gtc tac     240
Gln Gly Lys Ala Thr Leu Thr Ala Asp Lys Ser Ser Lys Thr Val Tyr
65                  70                  75                  80

atg cat ctg tct tcc ttg gca tct gag gat agc gct gtc tat tac tgt     288
Met His Leu Ser Ser Leu Ala Ser Glu Asp Ser Ala Val Tyr Tyr Cys
```

                              85                        90                        95
```
gct agg ggg gac tac tat ggg tca aat tcc ctg gat tac tgg ggc cag       336
Ala Arg Gly Asp Tyr Tyr Gly Ser Asn Ser Leu Asp Tyr Trp Gly Gln
            100                 105                 110

ggc acc agt gtc acc gtg agc agc                                        360
Gly Thr Ser Val Thr Val Ser Ser
        115                 120
```

<210> 66
<211> 120
<212> PRT
<213> Homo sapiens

<400> 66

```
Gln Val Gln Leu Val Gln Ser Gly Ala Glu Val Ala Lys Pro Gly Thr
1               5               10              15

Ser Val Lys Leu Ser Cys Lys Ala Ser Gly Tyr Thr Phe Thr Asp Tyr
            20              25              30

Trp Met Gln Trp Val Lys Gln Arg Pro Gly Gln Gly Leu Glu Trp Ile
            35              40              45

Gly Thr Ile Tyr Pro Gly Asp Gly Asp Thr Gly Tyr Ala Gln Lys Phe
        50              55              60

Gln Gly Lys Ala Thr Leu Thr Ala Asp Lys Ser Ser Lys Thr Val Tyr
65              70              75              80

Met His Leu Ser Ser Leu Ala Ser Glu Asp Ser Ala Val Tyr Tyr Cys
            85              90              95

Ala Arg Gly Asp Tyr Tyr Gly Ser Asn Ser Leu Asp Tyr Trp Gly Gln
            100                 105                 110

Gly Thr Ser Val Thr Val Ser Ser
        115                 120
```

<210> 67
<211> 324
<212> DNA
<213> Homo sapiens

<220>
<221> CDS
<222> (1)..(324)

<400> 67
```
gac acc gtg atg acc cag tcc ccc tcc acc atc tcc acc tct gtg ggc        48
Asp Thr Val Met Thr Gln Ser Pro Ser Thr Ile Ser Thr Ser Val Gly
1               5               10              15

gac cgg gtg tcc atc acc tgt aag gcc tcc cag gtg gtg ggc tcc gcc        96
Asp Arg Val Ser Ile Thr Cys Lys Ala Ser Gln Val Val Gly Ser Ala
            20              25              30
```

```
gtg gcc tgg tat cag cag aag cct ggc cag tcc cct aag ctg ctg atc      144
Val Ala Trp Tyr Gln Gln Lys Pro Gly Gln Ser Pro Lys Leu Leu Ile
        35              40              45

tac tgg gcc tcc acc cgg cat acc ggc gtg cct gac cgg ttc acc ggc      192
Tyr Trp Ala Ser Thr Arg His Thr Gly Val Pro Asp Arg Phe Thr Gly
    50              55              60

tcc ggc agc ggc acc gac ttc acc ctg acc atc tcc aac gtg cag tcc      240
Ser Gly Ser Gly Thr Asp Phe Thr Leu Thr Ile Ser Asn Val Gln Ser
65              70              75              80

gac gac ctg gcc gac tac ttc tgc cag cag tac aac tcc tac cct tac      288
Asp Asp Leu Ala Asp Tyr Phe Cys Gln Gln Tyr Asn Ser Tyr Pro Tyr
                85              90              95

acc ttt ggc ggc gga aca aag ctg gag atc aag cgt                      324
Thr Phe Gly Gly Gly Thr Lys Leu Glu Ile Lys Arg
            100             105
```

<210> 68
<211> 108
<212> PRT
<213> Homo sapiens

<400> 68

```
Asp Thr Val Met Thr Gln Ser Pro Ser Thr Ile Ser Thr Ser Val Gly
1               5               10              15

Asp Arg Val Ser Ile Thr Cys Lys Ala Ser Gln Val Val Gly Ser Ala
            20              25              30

Val Ala Trp Tyr Gln Gln Lys Pro Gly Gln Ser Pro Lys Leu Leu Ile
        35              40              45

Tyr Trp Ala Ser Thr Arg His Thr Gly Val Pro Asp Arg Phe Thr Gly
    50              55              60

Ser Gly Ser Gly Thr Asp Phe Thr Leu Thr Ile Ser Asn Val Gln Ser
65              70              75              80

Asp Asp Leu Ala Asp Tyr Phe Cys Gln Gln Tyr Asn Ser Tyr Pro Tyr
                85              90              95

Thr Phe Gly Gly Gly Thr Lys Leu Glu Ile Lys Arg
            100             105
```

<210> 69
<211> 324
<212> DNA
<213> Homo sapiens

<220>
<221> CDS
<222> (1)..(324)

<400> 69
```

```
gac acc gtg atg acc cag tcc ccc tcc tcc atc tcc acc tcc atc ggc          48
Asp Thr Val Met Thr Gln Ser Pro Ser Ser Ile Ser Thr Ser Ile Gly
1               5                   10                  15

gac cgg gtg tcc atc acc tgt aag gcc tcc cag gtg gtg ggc tcc gcc          96
Asp Arg Val Ser Ile Thr Cys Lys Ala Ser Gln Val Val Gly Ser Ala
                20                  25                  30

gtg gcc tgg tat cag cag aag cct ggc cag tcc cct aag ctg ctg atc        144
Val Ala Trp Tyr Gln Gln Lys Pro Gly Gln Ser Pro Lys Leu Leu Ile
            35                  40                  45

tac tgg gcc tcc acc cgg cat acc ggc gtg cct gcc cgg ttc acc ggc        192
Tyr Trp Ala Ser Thr Arg His Thr Gly Val Pro Ala Arg Phe Thr Gly
        50                  55                  60

tcc ggc agc ggc acc gac ttc acc ctg acc atc tcc aac gtg cag tcc        240
Ser Gly Ser Gly Thr Asp Phe Thr Leu Thr Ile Ser Asn Val Gln Ser
65                  70                  75                  80

gag gac ctg gcc gac tac ttc tgc cag cag tac aac tcc tac cct tac        288
Glu Asp Leu Ala Asp Tyr Phe Cys Gln Gln Tyr Asn Ser Tyr Pro Tyr
                85                  90                  95

acc ttt ggc ggc gga aca aag ctg gag atc aag cgt                        324
Thr Phe Gly Gly Gly Thr Lys Leu Glu Ile Lys Arg
                100                 105
```

```
<210>   70
<211>   108
<212>   PRT
<213>   Homo sapiens

<400>   70

Asp Thr Val Met Thr Gln Ser Pro Ser Ser Ile Ser Thr Ser Ile Gly
1               5                   10                  15

Asp Arg Val Ser Ile Thr Cys Lys Ala Ser Gln Val Val Gly Ser Ala
                20                  25                  30

Val Ala Trp Tyr Gln Gln Lys Pro Gly Gln Ser Pro Lys Leu Leu Ile
            35                  40                  45

Tyr Trp Ala Ser Thr Arg His Thr Gly Val Pro Ala Arg Phe Thr Gly
        50                  55                  60

Ser Gly Ser Gly Thr Asp Phe Thr Leu Thr Ile Ser Asn Val Gln Ser
65                  70                  75                  80

Glu Asp Leu Ala Asp Tyr Phe Cys Gln Gln Tyr Asn Ser Tyr Pro Tyr
                85                  90                  95

Thr Phe Gly Gly Gly Thr Lys Leu Glu Ile Lys Arg
                100                 105
```

```
<210>   71
<211>   351
<212>   DNA
<213>   Homo sapiens
```

```
<220>
<221>   CDS
<222>   (1)..(351)

<400>   71
gag gtg cag ctg gtg gag tct ggc ggc gga ctg gtg aag cct ggc ggc    48
Glu Val Gln Leu Val Glu Ser Gly Gly Gly Leu Val Lys Pro Gly Gly
1               5                   10                  15

tcc ctg agg ctg tcc tgt gag gcc tcc ggc ttc acc ttc tcc tcc tac    96
Ser Leu Arg Leu Ser Cys Glu Ala Ser Gly Phe Thr Phe Ser Ser Tyr
            20                  25                  30

acc ctg tcc tgg gtg agg cag acc cct ggc aag ggc ctg gag tgg gtg    144
Thr Leu Ser Trp Val Arg Gln Thr Pro Gly Lys Gly Leu Glu Trp Val
        35                  40                  45

gcc acc atc tcc atc ggc ggc agg tac acc tac tac cct gac tcc gtg    192
Ala Thr Ile Ser Ile Gly Gly Arg Tyr Thr Tyr Tyr Pro Asp Ser Val
    50                  55                  60

aag ggc cgg ttc acc atc tcc cgg gac aac gcc aag aac acc ctg tac    240
Lys Gly Arg Phe Thr Ile Ser Arg Asp Asn Ala Lys Asn Thr Leu Tyr
65                  70                  75                  80

ctg cag atg aac tcc ctg aag tcc gag gac acc gcc atg tac tac tgt    288
Leu Gln Met Asn Ser Leu Lys Ser Glu Asp Thr Ala Met Tyr Tyr Cys
                85                  90                  95

acc cgg gac ttc aac ggc tac tcc gac ttc tgg ggc cag ggc acc aca    336
Thr Arg Asp Phe Asn Gly Tyr Ser Asp Phe Trp Gly Gln Gly Thr Thr
            100                 105                 110

ctg acc gtg tcc tcc    351
Leu Thr Val Ser Ser
        115


<210>   72
<211>   117
<212>   PRT
<213>   Homo sapiens

<400>   72

Glu Val Gln Leu Val Glu Ser Gly Gly Gly Leu Val Lys Pro Gly Gly
1               5                   10                  15


Ser Leu Arg Leu Ser Cys Glu Ala Ser Gly Phe Thr Phe Ser Ser Tyr
            20                  25                  30


Thr Leu Ser Trp Val Arg Gln Thr Pro Gly Lys Gly Leu Glu Trp Val
        35                  40                  45


Ala Thr Ile Ser Ile Gly Gly Arg Tyr Thr Tyr Tyr Pro Asp Ser Val
    50                  55                  60


Lys Gly Arg Phe Thr Ile Ser Arg Asp Asn Ala Lys Asn Thr Leu Tyr
65                  70                  75                  80


Leu Gln Met Asn Ser Leu Lys Ser Glu Asp Thr Ala Met Tyr Tyr Cys
```

34

```
                    85                    90                    95
```

Thr Arg Asp Phe Asn Gly Tyr Ser Asp Phe Trp Gly Gln Gly Thr Thr
            100                    105                   110

Leu Thr Val Ser Ser
       115

```
<210>  73
<211>  36
<212>  DNA
<213>  Mus sp.

<400>  73
ggaggatcca tagacagatg ggggtgtcgt tttggc                          36


<210>  74
<211>  32
<212>  DNA
<213>  Mus sp.

<400>  74
ggaggatccc ttgaccaggc atcctagagt ca                              32


<210>  75
<211>  32
<212>  DNA
<213>  Mus sp.


<220>
<221>  misc_feature
<222>  (1)..(32)
<223>  mixed bases are defined as follows: H=A+T+C, S=G+C, Y=C+T,  K=
       G+T,  M=A+C,  R=A+G,  W=A+T,  V = A+C+G, N = A+C+G+T

<400>  75
cttccggaat tcsargtnma gctgsagsag tc                              32


<210>  76
<211>  35
<212>  DNA
<213>  Mus sp.


<220>
<221>  misc_feature
<222>  (1)..(35)
<223>  mixed bases are defined as follows: H=A+T+C, S=G+C, Y=C+T,  K=
       G+T,  M=A+C,  R=A+G,  W=A+T,  V = A+C+G, N = A+C+G+T

<400>  76
cttccggaat tcsargtnma gctgsagsag tcwgg                           35


<210>  77
<211>  31
<212>  DNA
<213>  Mus sp.


<220>
```

```
<221>  misc_feature
<222>  (1)..(31)
<223>  mixed bases are defined as follows: H=A+T+C, S=G+C, Y=C+T,  K=
       G+T,  M=A+C,  R=A+G,  W=A+T,  V = A+C+G, N = A+C+G+T

<400>  77
ggagctcgay attgtgmtsa cmcarwctmc a                                      31


<210>  78
<211>  46
<212>  DNA
<213>  Mus sp.

<400>  78
tatagagctc aagcttggat ggtgggaaga tggatacagt tggtgc                     46


<210>  79
<211>  21
<212>  DNA
<213>  Mus sp.

<400>  79
atggagtcac agattcaggt c                                                 21


<210>  80
<211>  32
<212>  DNA
<213>  Mus sp.

<400>  80
ttttgaattc cagtaacttc aggtgtccac tc                                     32
```

**Claims**

1. A pharmaceutical combination comprising an antibody specifically recognizing CD38 and at least cyclophosphamide, wherein said antibody is capable of killing a CD38$^+$ cell by apoptosis, antibody-dependent cell-mediated cytotoxicity (ADCC), and complement-dependent cytotoxicity (CDC).

2. The combination of claim 1, wherein said antibody is a humanized antibody.

3. The combination of claim 2 wherein said antibody comprises one or more complementarity-determining region having an amino acid sequence selected from the group consisting of SEQ ID NOS: 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, and 36.

4. The combination of claim 3 wherein said antibody comprises at least one heavy chain and at least one light chain, wherein said heavy chain has an amino acid sequence represented by SEQ ID NO: 66 and said heavy chain comprises three sequential complementarity-determining regions having amino acid sequences represented by SEQ ID NOS: 13, 14, and 15, and wherein said light chain has an amino acid sequence selected from the group of SEQ ID NOS: 62 and 64, and said light chain comprises three sequential complementarity-determining regions having amino acid sequences represented by SEQ ID NOS: 16, 17, and 18.

5. The combination of claim 3 wherein said antibody comprises at least one heavy chain and at least one light chain, wherein said heavy chain has an amino acid sequence represented by SEQ ID NO: 72 and said heavy chain comprises three sequential complementarity-determining regions having amino acid sequences represented by SEQ ID NOS: 25, 26, and 27, and wherein said light chain has an amino acid sequence selected from the group of SEQ ID NOS: 68 and 70, and said light chain comprises three sequential complementarity-determining regions having amino acid sequences represented by SEQ ID NOS: 28, 29, and 30.

6. Use of an antibody specifically recognizing CD38 for the preparation of a pharmaceutical combination according to claim 1 for the manufacture of a medicament for the treatment of cancer.

7. The use of claim 6, wherein said antibody is a humanized antibody.

8. The use of claim 7 wherein said antibody comprises one or more complementarity-determining region having an amino acid sequence selected from the group consisting of SEQ ID NOS: 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, and 36.

9. The use of claim 8 wherein said antibody comprises at least one heavy chain and at least one light chain, wherein said heavy chain has an amino acid sequence represented by SEQ ID NO: 66 and said heavy chain comprises three sequential complementarity-determining regions having amino acid sequences represented by SEQ ID NOS: 13, 14, and 15, and wherein said light chain has an amino acid sequence selected from the group of SEQ ID NOS: 62 and 64, and said light chain comprises three sequential complementarity-determining regions having amino acid sequences represented by SEQ ID NOS: 16, 17, and 18.

10. The use of claim 8 wherein said antibody comprises at least one heavy chain and at least one light chain, wherein said heavy chain has an amino acid sequence represented by SEQ ID NO: 72 and said heavy chain comprises three sequential complementarity-determining regions having amino acid sequences represented by SEQ ID NOS: 25, 26, and 27, and wherein said light chain has an amino acid sequence selected from the group of SEQ ID NOS: 68 and 70, and said light chain comprises three sequential complementarity-determining regions having amino acid sequences represented by SEQ ID NOS: 28, 29, and 30.

Europäisches
Patentamt

European
Patent Office

Office européen
des brevets

## EUROPEAN SEARCH REPORT

**Application Number**

EP 08 29 1119

### DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | EP 1 914 242 A (SANOFI AVENTIS [FR]) 23 April 2008 (2008-04-23) * page 3, line 47 - line 51 * * page 26, line 19 * * page 26, line 25 - line 33 * | 1-10 | INV. A61K39/00 A61K31/00 A61P35/00 |
| X | WO 2006/099875 A (GENMAB AS [DK]; WEERS MICHEL DE [NL]; GRAUS YVO [NL]; OPRINS JUDITH [N) 28 September 2006 (2006-09-28) * page 176, line 15 - line 29 * * page 168, line 10 - page 172, line 8 * | 1-10 | |
| X | WO 99/62526 A (MAYO FOUNDATION [US]; LUST JOHN A [US]; DONOVAN KATHLEEN A [US]) 9 December 1999 (1999-12-09) * claims 1,2 * * page 9, line 24 - page 10, line 2 * | 1-10 | |
| A | ELLIS JONATHAN H ET AL: "Engineered anti-CD38 monoclonal antibodies for immunotherapy of multiple myeloma" JOURNAL OF IMMUNOLOGY, AMERICAN ASSOCIATION OF IMMUNOLOGISTS, US, vol. 155, no. 2, 1 January 1995 (1995-01-01), pages 925-937, XP002146232 ISSN: 0022-1767 * the whole document * | 1-10 | TECHNICAL FIELDS SEARCHED (IPC) A61K |

-/--

The present search report has been drawn up for all claims

| Place of search | Date of completion of the search | Examiner |
|---|---|---|
| Munich | 25 May 2009 | Rodrigo-Simón, Ana |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

Europäisches
Patentamt
European
Patent Office
Office européen
des brevets

# EUROPEAN SEARCH REPORT

**Application Number**

EP 08 29 1119

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | PEIPP M ET AL: "FULLY HUMAN CD38 ANTIBODIES EFFICIENTLY TRIGGER ADCC AND CDC OF MULTIPLE MYELOMA AND PLASMA CELL LEUKEMIA CELLS" INTERNET CITATION, [Online] XP002473215 Retrieved from the Internet: URL:http://www.genmab.com/upload/poster_ash_2005_monday_05-12-2005.pdf> [retrieved on 2008-03-18] * the whole document * | 1-10 | |
| A | PEIPP M ET AL: "FULLY HUMAN CD38 ANTIBODIES EFFICIENTLY TRIGGER ADCC OF MULTIPLE MYELOMA CELL LINES AND PRIMARY TUMOR CELLS" BLOOD, AMERICAN SOCIETY OF HEMATOLOGY, US, vol. 106, no. 11, PART 01, 1 November 2005 (2005-11-01), page 944A,ABSTRACTNO.3377, XP009069301 ISSN: 0006-4971 * the whole document * & PEIPP M ET AL: "Fully human CD38 antibodies efficiently trigger ADCC of multiple myeloma cell lines and primary tumor cells" BIOSIS,, 1 January 1900 (1900-01-01), XP002389878 | 1-10 | |
| A | URUSHIZAKI I: "RECENT VIEW OF TUMOR DORMANCY THERAPY" BIOTHERAPY, KANI SHOBO, TOKYO, JP, vol. 17, no. 4, 1 July 2003 (2003-07-01), pages 331-338, XP009025509 ISSN: 0914-2223 * abstract * | 1-10 | TECHNICAL FIELDS SEARCHED (IPC) |
| A | WO 2004/026337 A (ISIS INNOVATION [GB]; CERUNDOLO VINCENZO [GB]; HERMANS IAN FRANCIS [GB]) 1 April 2004 (2004-04-01) * page 1, line 1 - page 5, line 10 * | 1-10 | |

The present search report has been drawn up for all claims

| Place of search | Date of completion of the search | Examiner |
|---|---|---|
| Munich | 25 May 2009 | Rodrigo-Simón, Ana |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
& : member of the same patent family, corresponding document

# EP 2 191 843 A1

## ANNEX TO THE EUROPEAN SEARCH REPORT
## ON EUROPEAN PATENT APPLICATION NO.

EP 08 29 1119

This annex lists the patent family members relating to the patent documents cited in the above-mentioned European search report.
The members are as contained in the European Patent Office EDP file on
The European Patent Office is in no way liable for these particulars which are merely given for the purpose of information.

25-05-2009

| Patent document cited in search report | | Publication date | Patent family member(s) | | Publication date |
|---|---|---|---|---|---|
| EP 1914242 | A | 23-04-2008 | AR | 063488 A1 | 28-01-2009 |
| | | | AU | 2007311511 A1 | 24-04-2008 |
| | | | CL | 29952007 A1 | 16-05-2008 |
| | | | WO | 2008047242 A2 | 24-04-2008 |
| | | | UY | 30655 A1 | 31-05-2008 |
| WO 2006099875 | A | 28-09-2006 | AU | 2006226733 A1 | 28-09-2006 |
| | | | CA | 2602375 A1 | 28-09-2006 |
| | | | EP | 1866338 A1 | 19-12-2007 |
| | | | JP | 2008533977 T | 28-08-2008 |
| | | | KR | 20070116137 A | 06-12-2007 |
| WO 9962526 | A | 09-12-1999 | AU | 770718 B2 | 26-02-2004 |
| | | | AU | 4418599 A | 20-12-1999 |
| | | | CA | 2329940 A1 | 09-12-1999 |
| | | | EP | 1085882 A2 | 28-03-2001 |
| | | | JP | 2003524587 T | 19-08-2003 |
| WO 2004026337 | A | 01-04-2004 | AU | 2003269133 A1 | 08-04-2004 |
| | | | CA | 2499628 A1 | 01-04-2004 |
| | | | EP | 1545592 A1 | 29-06-2005 |

EPO FORM P0459

For more details about this annex : see Official Journal of the European Patent Office, No. 12/82

40

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 2008047242 A
- WO 9222653 A
- US 5639641 A
- EP 0239400 A
- WO 9109967 A
- US 5530101 A
- US 5585089 A
- EP 0592106 A
- EP 0519596 A
- US 5565332 A
- US 2008074381 W
- US 4444887 A
- US 4716111 A
- US 5545806 A
- US 5814318 A
- WO 9846645 A
- WO 9850433 A
- WO 9824893 A
- WO 9816654 A
- WO 9634096 A
- WO 9633735 A
- WO 9110741 A

**Non-patent literature cited in the description**

- **Padlan E. A.** *Molecular Immunology,* 1991, vol. 28 (4/5), 489-498
- **Studnicka G. M. et al.** *Protein Engineering,* 1994, vol. 7 (6), 805-814
- **Roguska M.A. et al.** *PNAS,* 1994, vol. 91, 969-973
- **T.H. Corbett et al.** *Cancer,* 1977, vol. 40, 2660-2680
- Cancer Drug Development, Part B, Methods. **F.M. Schabel et al.** Cancer Research. Academic Press Inc, 1979, vol. 17, 3-51